# EUROPÄISCHE PATENTSCHRIFT

(11) **EP 2 830 526 B1**
(45) Veröffentlichungstag und Bekanntmachung des Hinweises auf die Patenterteilung: **15.05.2019**
(21) Anmeldenummer: 13714616.3
(22) Anmeldetag: 28.03.2013
(51) Int. Cl.: A61B 34/20

(54) **MEDIZINISCHES NAVIGATIONSSYSTEM MIT DRAHTLOS VERBUNDENEM, BERÜHRUNGSEMPFINDLICHEM BILDSCHIRM**
MEDICAL NAVIGATION SYSTEM WITH WIRELESSLY CONNECTED, TOUCH-SENSITIVE SCREEN
SYSTÈME DE NAVIGATION MÉDICALE MUNI D'UN ÉCRAN TACTILE CONNECTÉ SANS FIL

(30) Priorität: 29.03.2012 DE 102012205165
(43) Veröffentlichungstag der Anmeldung: 04.02.2015
(73) Patentinhaber: Fiagon AG Medical Technologies, 16761 Hennigsdorf (DE)
(72) Erfinder: KRÜGER, Timo, 13465 Berlin (DE); MUCHA, Dirk, 13467 Berlin (DE); ROSE, Andreas, 16727 Oberkrämer (DE); BRAUN, Hannes, 8043 Graz (AT)
(74) Vertreter: Eisenführ Speiser
(86) Internationale Anmeldenummer: PCT/EP2013/056802
(87) Internationale Veröffentlichungsnummer: WO 2013/144334

(56) Entgegenhaltungen:
- EP-A1- 1 915 962
- EP-A1- 2 179 703
- US-A1- 2004 263 535
- US-A1- 2005 020 909
- US-A1- 2008 077 158
- OLWAL ALEX ET AL: "Design and Evaluation of Interaction Technology for Medical Team Meetings", 5 September 2011 (2011-09-05), MEDICAL IMAGE COMPUTING AND COMPUTER-ASSISTED INTERVENTION - MICCAI 2015 : 18TH INTERNATIONAL CONFERENCE, MUNICH, GERMANY, OCTOBER 5-9, 2015; PROCEEDINGS; [LECTURE NOTES IN COMPUTER SCIENCE; LECT.NOTES COMPUTER], SPRINGER INTERNATIONAL PUBLISHING, CH, XP047324803, ISSN: 0302-9743 ISBN: 978-3-030-00888-8
- Alex Olwal: "HybridSurface: Multi-user, multi-device, remote collaboration on Vimeo", , 15 October 2011 (2011-10-15), XP055513174, Retrieved from the Internet: URL:https://vimeo.com/30581634 [retrieved on 2018-10-08]

## Beschreibung

Die Dokumente US2008077158 A1, EP1915962 A1, US2004263535 A1, US2005020909 A1 und EP2179703 A1 offenbaren Medizinische Navigationssysteme mit drahtlos verbundenen Bedieneinheiten.

Die Publikation OLWAL ALEX ET AL: "Design and Evaluation of Interaction Technology for Medical Team Meetings", 5. September 2011 (2011-09-05), MEDICAL IMAGE COMPUTING AND COMPUTER-ASSISTED INTERVENTION - MICCAI 2015 : 18TH INTERNATIONAL CONFERENCE, MUNICH, GERMANY, OCTOBER 5-9, 2015; PROCEEDINGS; [LECTURE NOTES IN COMPUTER SCIENCE; LECT.NOTES COMPUTER], SPRINGER INTERNATIONAL PUBLISHING, CH, offenbart ein System zur multidisziplinären Begutachtung von medizinischen Bilddaten mithilfe eines Systems welches mehrere handgehaltene Eingabegeräte mit Bildschirmen nutzt. Die Erfindung betrifft ein medizinisches System mit einem Instrument, einer Lageerfassungseinrichtung für das Instrument und einer Datenverarbeitungs- und Bilderzeugungseinrichtung.

Mit einem derartigen medizinischen System kann beispielsweise die Position und Orientierung eines medizinischen Instrumentes erfasst werden und die so erfassten Positionsdaten können beispielsweise einem tomographischen Abbild eines Objektes oder Körperteils zugeordnet werden. Die Datenverarbeitungs- und Bilderzeugungseinrichtung ist dann dazu in der Lage, aus aktuell aufgenommenen oder gespeicherten, ein jeweiliges Körperteil repräsentierenden Daten ein Bild eines Körperteils zu generieren, das eine Ansicht des Körperteils zusammen mit einer Darstellung der Position und Orientierung des Instrumentes derart wiedergibt, dass ein Betrachter die Position und Orientierung des Instrumentes in dem Körperteil dem Bild des Körperteils entnehmen kann. Die dazu von der Lageerfassungseinrichtung aufgenommenen Positionsdaten geben typischerweise eine Position und Orientierung eines jeweiligen Instruments bezüglich eines Referenzkoordinatensystems wieder und können daher von der Datenverarbeitungs- und Bilderzeugungseinrichtung in ein den Bilddaten zugrundeliegendes Koordinatensystem transformiert werden. Derartige medizinische Systeme sind grundsätzlich bekannt und dienen der sogenannten Navigation chirurgischer Instrumente, die typischerweise handgeführt sind. Ein Operateur hat somit die Möglichkeit, in einer, beispielsweise tomographischen Wiedergabe seines Patienten auch die Position des von ihm geführten Instrumentes zu erkennen und so das Instrument gezielt zu einem Zielgebiet oder einer Zielposition zu führen. Die eingesetzten Lageerfassungssysteme können beispielsweise optische, ultraschallgestützte oder elektromagnetische Lageerfassungseinrichtungen sein. So sind beispielsweise elektromagnetische Lageerfassungseinrichtungen bekannt, bei denen ein Feldgenerator ein elektromagnetisches Wechselfeld erzeugt und am Instrument Positionssensoren vorgesehen sind, die Spulen aufweisen. Durch das elektromagnetische Wechselfeld des Generators werden in den Spulen Ströme induziert, die von der Ausrichtung einer jeweiligen Spule zum elektromagnetischen Wechselfeld abhängen. Wenn ein bewegliches Instrument mit derartigen Positionssensoren in Form von Sensorspulen ausgestattet ist, ist es möglich, Ort und Lage des Instrumentes relativ zu einem Referenzsensor, der beispielsweise ebenfalls Spulen aufweisen kann, zu bestimmen. Dabei ist der Referenzsensor beispielsweise als Patientenlokalisator fest mit einem Körperteil eines Patienten (oder auch einem anderen Objekt) verbunden.

Für eine Navigation in Körperteilen eines Patienten wird typischerweise die Position und Orientierung eines Instrumentes mit einer derartigen Lageerfassungseinrichtung erfasst und die Position des Instrumentes anschließend in tomographisch gewonnenen Schnittbildern des Körperteils angezeigt. Damit dies funktioniert, müssen die Positionsdaten, die der Positionssensor des Instrumentes liefert, in Koordinaten des tomographischen Abbildes des Patienten übertragen werden. Die Position des Instrumentes und vorzugsweise auch dessen Orientierung kann dann in Schnittbildern oder auch perspektivischen Darstellungen des Körperteils eines Patienten dargestellt werden.

Für den Operateur ist es hilfreich, wenn er hierfür die jeweils günstigste Darstellung auswählen kann, beispielsweise unterschiedliche Schnittebenen auswählen kann, um eine jeweils ihm hilfreiche Darstellung wählen zu können. Deshalb ist es vorgesehen, derartige Lageerfassungseinrichtungen bzw. Datenverarbeitungs- und Bilderzeugungseinrichtungen mit Eingabemitteln auszustatten, die beispielsweise die Auswahl einer Darstellungsform erlauben.

Der Erfindung liegt die Aufgabe zugrunde, ein derartiges System vor allem hinsichtlich der Handhabbarkeit durch den Operateur zu verbessern.

Erfindungsgemäß wird diese Aufgabe durch ein medizinisches System mit einem Instrument, einer Datenverarbeitungs- und Bilderzeugungseinrichtung und einer Bilddarstellungs- und Steuereinheit gelöst. Von diesen ist die Lageerfassungseinrichtung ausgebildet, eine Position und Orientierung des Instruments bezüglich eines Referenzkoordinatensystems zu erfassen. Die Datenverarbeitungs- und Bilderzeugungseinrichtung ist ausgebildet, aus aktuell aufgenommenen oder gespeicherten, ein Körperteil repräsentierenden Daten ein Bild eines Körperteils zu erzeugen, das eine Ansicht des Körperteils zusammen mit einer Darstellung der Position und vorzugsweise auch der Orientierung des Instrumentes derart wiedergibt, dass ein Betrachter die Position und Orientierung des Instrumentes in dem Körperteil dem Bild des Körperteils entnehmen kann. Erfindungsgemäß ist hierbei die Bilddarstellungs- und Steuereinheit drahtlos mit der Datenverarbeitungs- und Bilderzeugungseinrichtung verbunden und besitzt eine Anzeige- und Eingabeeinheit mit einer geschlossenen Oberfläche. Auf der geschlossenen Oberfläche der Anzeige- und Eingabeeinheit sind im Betrieb der Bilddarstellungs- und Steuereinheit Steuerelemente sowie eine jeweilige Ansicht des Körperteils zusammen mit einer Darstellung der Position und vorzugsweise auch Orientierung des Instrumentes dargestellt. Die Bilddarstellungs- und Steuereinheit ist dazu ausgebildet, über angezeigte Steuerelemente Benutzereingaben entgegenzunehmen und in Abhängigkeit jeweils entgegengenommener Benutzereingaben Steuersignale an die Datenverarbeitungs- und Bilderzeugungseinrichtung zu senden.

Die Anzeige- und Eingabeeinheit kann beispielsweise ein Touchscreen sein, also ein berührungsempfindlicher Bildschirm, der auch Eingaben über den Bildschirm erlaubt. Dadurch dass eine derartige Anzeige- und Eingabeeinheit geschlossen ist, kann die Anzeige- und Eingabeeinheit auch leicht im Operationssaal eingesetzt werden, da sie nicht so anfällig für Kontamination ist.

Da die Bilddarstellungs- und Steuereinheit außerdem drahtlos mit der Datenverarbeitungs- und Bilderzeugungseinrichtung verbunden ist, kann die Bilddarstellungs- und Steuereinheit vom Operateur an der jeweils günstigsten Stelle abgelegt oder installiert werden oder auch freihand bedient werden. So kann der Operateur direkt vom Operationsort Steuereingaben machen oder Abbildungen auf der Bilddarstellungs- und Steuereinheit unmittelbar mit dem vergleichen, was er bei einem Patienten vor Augen hat.

Um insbesondere letzten Aspekt zu erleichtern, ist das medizinische System vorzugsweise so ausgebildet, dass die Anzeige- und Steuereinheit eine jeweilige Ansicht eines Körperteils im Maßstab 1:1 darstellt, so dass Entfernungen in einer dargestellten Schnittebene eines Körperteils eine Entfernung entlang der Schnittebene in dem Körperteil selbst wenigstens annähernd identisch sind. Diese erlaubt nicht nur den Vergleich dessen, was der Operateur beim Patienten vor Augen hat mit entsprechenden Abbildungen.

Außerdem erlaubt es dem Operateur, sein Instrument besonders genau zu führen, da die der Bilddarstellung auf der Darstellungs- und Steuereinheit zu entnehmenden Entfernungen genau denen entsprechen, um die er gegebenenfalls das Instrument bewegen muss, um beispielsweise eine bestimmte Zielposition zu erreichen.

In diesem Zusammenhang ist es besonders bevorzugt, wenn die Datenverarbeitungs- und Bilderzeugungseinrichtung außerdem dazu ausgebildet ist, in einer jeweiligen Ansicht eines Körperteils neben einer jeweiligen Position und vorzugsweise auch eine Orientierung des Instrumentes sowie eine Markierung einer weiteren Position und/oder eines Zielgebietes oder Zielvolumens in dem Körperteil anzuzeigen.

Vorzugsweise ist die Datenverarbeitungs- und Bilderzeugungseinrichtung ausgebildet, auf unterschiedliche, ein jeweiliges Körperteil repräsentierende Daten zuzugreifen, beispielsweise auf computertomographisch gewonnene Daten oder auf magnetresonanztomographisch gewonnene Daten. Die Datenverarbeitungs- und Bilderzeugungseinrichtung ist außerdem entsprechend dazu ausgebildet, nach Wahl ein Bild zu erzeugen, das auf computertomographisch gewonnenen Daten basiert oder ein Bild, das auf magnetresonanztomographisch gewonnenen Daten basiert. Die Bilddarstellungs- und Steuereinheit ist in diesem Zusammenhang vorzugsweise dazu ausgebildet, eine oder mehrere Steuerelemente darzustellen, die der Auswahl zwischen verschiedenen, ein jeweiliges Körperteil repräsentierenden Daten und damit der entsprechenden Bilddarstellung dienen. Die Bilddarstellungs- und Steuereinheit ist dazu ausgebildet, bei Betätigung eines entsprechenden Steuerelementes ein Steuersignal zu erzeugen, das die Datenverarbeitungs- und Bilderzeugungseinrichtung dazu veranlasst, ein Bild auf Basis der entsprechend ausgewählten, ein Körperteil des Patienten repräsentierenden Daten zu generieren.

Außerdem ist die Datenverarbeitungs- und Bilderzeugungseinrichtung vorzugsweise dazu ausgebildet, ein angezeigtes Bild zu speichern, so dass es später wieder aufgerufen werden kann. Dies ist deshalb von Vorteil, da sich typischerweise die Position des Instrumentes im Körperteil und möglicherweise auch das Körperteil mit der Zeit verändern bzw. bewegen, so dass sich typischerweise ein aktuell wiedergegebenes Bild von einem zu früherem Zeitpunkt wiedergegebenen Bild unterscheidet. Deshalb kann es hilfreich sein, zu bestimmten Zeitpunkten ein Bild zu speichern, beispielsweise im Sinne eines Screenshots. Auch hierzu weist die Bilddarstellungs- und Steuereinheit vorzugsweise ein entsprechendes Steuerelement auf, bei dessen Betätigung die Bilddarstellungs- und Steuereinheit ein Steuersignal erzeugt, das die Datenverarbeitungs- und Bilderzeugungseinrichtung dazu veranlasst, ein bei Betätigung des Steuerelementes jeweils aktuell angezeigtes Bild zu speichern.

In ähnlicher Weise kann es wünschenswert sein, dass die Datenverarbeitungs- und Bilderzeugungseinrichtung ausgebildet ist, eine Bildfolge nach Art eines Films zu speichern. Auch um dieses auszulösen, weist die Bilddarstellungs- und Steuereinheit vorzugsweise ein entsprechendes Steuerelement auf, bei dessen Betätigung die Bilddarstellungs- und Steuereinheit ein Steuersignal erzeugt, das die Datenverarbeitungs- und Bilderzeugungseinrichtung dazu veranlasst, eine Bildfolge im Sinne eines Films aufzunehmen.

Gemäß einer besonders bevorzugten Ausführungsvariante ändert sich die Darstellung des Steuerelementes zum Auslösen des Aufnehmens einer Bildfolge, bei der Betätigung, die das Aufnehmen einer Bildfolge startet. Der Operateur kann so anhand der Darstellung des Steuerelementes selbst erkennen, dass gerade eine Bildfolge aufgenommen wird. Die Bilddarstellungs- und Steuereinrichtung ist vorzugsweise weiterhin dazu ausgebildet, dass ein zweites Betätigen des Steuerelementes für die Aufnahme von Bildfolgen die Aufnahme einer jeweiligen Bildfolge wieder stoppt. Vorzugsweise erzeugt die Bilddarstellungs- und Steuereinheit beim jeweils zweitmaligen Betätigen des Steuerelementes wieder die ursprüngliche Darstellung dieses Steuerelementes vor dem Auslösen der Aufnahme einer Bildfolge.

Die Erfindung soll nun anhand eines Ausführungsbeispiels mit Bezug auf die Figuren näher erläutert werden. Von diesen zeigt:
- Figur 1:: ein medizinisches System mit einem Instrument, einer Lageerfassungseinrichtung für dieses Instrument, einer Datenverarbeitungs- und Bilderzeugungseinrichtung sowie einer Bilddarstellungs- und Steuereinheit;
- Figur 2:: ein medizinisches Instrument und eine dazugehörige Lageerfassungseinrichtung mit einem Feldgenerator für ein elektromagnetisches Wechselfeld sowie einem patientenfesten Positionssensor;
- Figur 3:: ein Beispiel eines von der Datenverarbeitungs- und Bilderzeugungseinrichtung generierten und auf der Bilddarstellungs- und Steuereinheit dargestellten Bildes zusammen mit ebenfalls auf der Bilddarstellungs- und Steuereinheit dargestellten Steuerelementen; und
- Figur 4:: eine Abbildung von auf der Bilddarstellungs- und Steuereinheit dargestellten Steuerelementen.

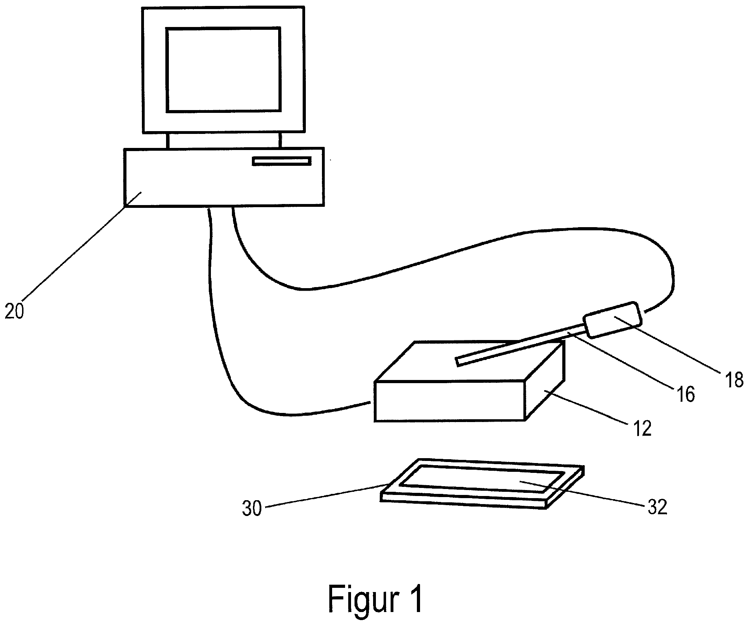

Figur 1 zeigt die Komponenten eines medizinischen Systems, nämlich ein Instrument 16, einen Feldgenerator 12 als Teil einer Lageerfassungseinrichtung und eine Datenverarbeitungs- und Bilderzeugungseinrichtung 20, die über Kabel 22 und 24 mit dem Feldgenerator 12 bzw. dem Instrument 16 verbunden ist. Außerdem zeigt Figur 1 eine Bilddarstellungs- und Steuereinheit 30, die leicht mit ein oder zwei Händen zu halten ist und die drahtlos mit der Datenverarbeitungs- und Bilderzeugungseinrichtung 20 verbunden ist.

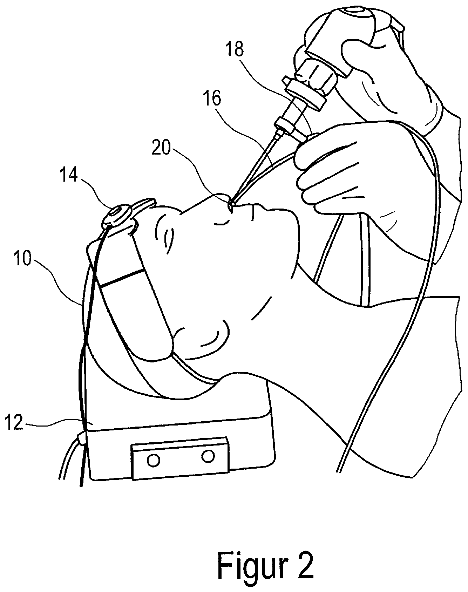

Fig. 2 zeigt skizzenhaft einen Kopf 10 eines Patienten in einer Seitenansicht. In der Nachbarschaft des Kopfes 10 befindet sich der Feldgenerator 12 zum Erzeugen eines elektromagnetischen Wechselfeldes, das im Bereich des Kopfes des Patienten zu erfassen ist. Am Kopf 10 des Patienten ist ein patientenfester Positionssensor als Referenzpositionssensor 14 bzw. Patientenlokalisator befestigt. Das bewegliche Instrument 16 besitzt ein proximales Ende mit einem Handgriff 18 und ein distales Ende mit einem Instrumentenpositionssensor. Der Referenzpositionssensor 14 und der Instrumentenpositionssensor weisen jeweils eine oder mehrere elektrische Spulen als Sensorspulen auf. Das von dem Feldgenerator 12 im Betrieb ausgehende elektromagnetische Wechselfeld induziert in den Spulen jeweils einen Strom, dessen Amplitude von der relativen Position und Ausrichtung einer jeweiligen Sensorspule zum Feldgenerator 12 abhängt. Auf diese Weise ist es in an sich bekannter Weise möglich, die relative Position und Ausrichtung (Orientierung) des Instrumentenpositionssensors in Bezug auf den Referenzpositionssensor zu bestimmen.

Um mittels eines Lageerfassungssystems erfasste Positionswerte eines Instrumentes für eine Instrumentennavigation in dem Sinne zu nutzen, dass eine jeweils aktuelle Position eines Instrumentes in beispielsweise tomographisch gewonnenen Schnittbildern eines Körperteils angezeigt wird, müssen die vom Positionssensor des Instrumentes gelieferten Positionswerte in ein einem tomographischen Abbild zugrundeliegendes Modellkoordinatensystem transformiert werden. Eine hierzu nötige Transformationsvorschrift wird in an sich bekannter Weise mittels eines Registrierverfahrens gewonnen. Dazu wird beispielsweise mit einem Zeige- oder Abtastinstrument eine Oberfläche eines Körperteils abgetastet, und dabei gewonnene Positionswerte werden mit möglichst kleinem Fehler auf einer von einem tomographischen oder topographischen Abbild abgeleitete Modelloberfläche übertragen. Die für diese möglichst genaue Übertragung erforderlichen Skalierungen, Rotationen und/oder Translationen oder dergleichen ergeben schließlich die Transformationsvorschrift.

Die Datenverarbeitungs- und Bilderzeugungseinrichtung 20 kann auch vom Operationsort entfernt sein, beispielsweise Teil eines zentralen Rechenzentrums.

Das Instrument 16 kann ein chirurgisches Instrument sein, oder ein Mikroskop, ein Endoskop oder ein anderes bewegliches Instrument. Das Instrument 16 muss dabei nicht unbedingt manuell beweglich sein, sondern kann auch robotergesteuert sein. Die Bilddarstellungs- und Steuereinheit 30 ist als autarke Einheit ausgebildet, die drahtlos wenigstens mit der Datenverarbeitungs- und Bilderzeugungseinrichtung 20 kommuniziert, aber zusätzlich auch beispielsweise mit dem Instrument 16 kommunizieren kann, um insbesondere das Instrument 16 mittels der Bilddarstellungs- und Steuereinheit 30 fernsteuern zu können. Wenn das Instrument 16 beispielsweise ein Endoskop ist, kann mittels der Bilddarstellungs- und Steuereinheit 30 die Ausrichtung der Endoskopoptik gesteuert werden, sofern diese ferngesteuert ausrichtbar ist. Falls das Instrument 16 ein Mikroskop ist, kann die Darstellungs- und Steuereinheit 30 ausgebildet sein, die Fokussteuerung des Mikroskops oder die Übersteuerung oder Einstellung der Autofokussteuerung des Mikroskops fernzusteuern.

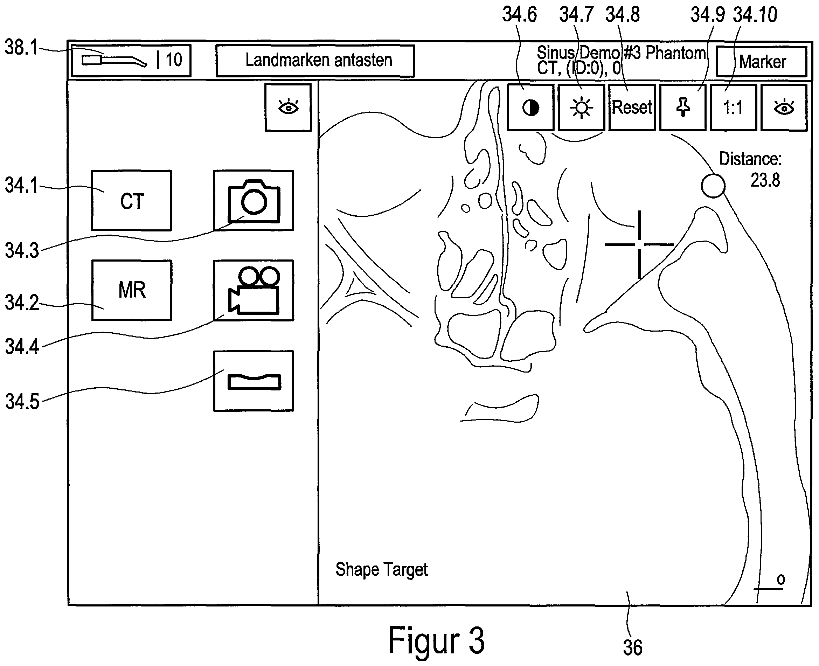

Wie der Figur 3 zu entnehmen ist, zeigt die Bilddarstellungs- und Steuereinheit 30 auf ihrer Anzeige- und Eingabeeinheit 32 sowohl wenigstens eine Ansicht 36 eines Körperteils an als auch ein oder mehrere Steuerelemente 34 sowie Informationsicons 38. Wie bereits beschrieben, kann die Ansicht 36 eines Körperteils eine von der Datenverarbeitungs- und Bilderzeugungseinheit 20 aus beispielsweise computertomographisch oder magnetresonanztomographisch gewonnenen Daten eines Körperteils erzeugt sein. Zusätzlich oder alternativ kann die dargestellte Ansicht auch eine jeweils aktuelle Endoskop- oder Mikroskopaufnahme sein. Vorzugsweise werden sowohl aus tomographisch gewonnenen Daten angezeigte Ansichten als auch mittels eines optischen Instruments wie eines Endoskops oder eines Mikroskops aufgenommenen Ansichten gleichzeitig angezeigt. Die Anzeige der Ansicht eines jeweiligen Körperteils erfolgt vorzugsweise im Stab 1:1, so dass Entfernungen auf der Ebene der Anzeige- und Eingabeeinheit 32 der Bilddarstellungs- und Steuereinheit 30 Entfernungen in der entsprechenden Ebene des Körperteils entsprechen.

In den jeweiligen Ansichten eines Körperteils werden sowohl vorzugsweise die Position eines jeweiligen Instruments 16 als auch beispielsweise Planungsinformationen angezeigt, die Ergebnisse einer vorangegangenen Operationsplanung sind. Solche Planungsinformationen können beispielsweise Markierungen eines Zielgebietes oder eines Zielvolumens in der jeweils dargestellten Ansicht 36 des Körperteils sein. Andere Planungsinformationen können die Position und Ausrichtung von Implantaten wie z.B. Schrauben sein. Wenn diese in der Ansicht 36 des Körperteils dargestellt sind, kann ein Operateur das reale Implantat während der Operation genau an der dargestellten Planungsinformation in der Ansicht 36 des Körperteils ausrichten.

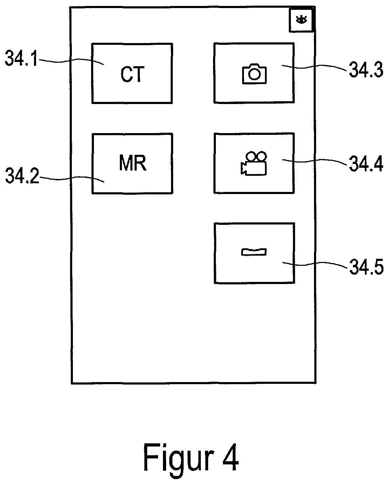

Den Figuren 3 und 4 sind beispielhaft auf der Anzeige- und Eingabeeinheit 32 angezeigte Steuerelemente 34 zu entnehmen. Ein erstes Steuerelement 34.1 und ein zweites Steuerelement 34.2 dienen beispielsweise der Auswahl der ein jeweiliges Körperteil repräsentierenden Daten.

So kann ein Nutzer durch Betätigen des ersten Steuerelementes 34.1 bewirken, dass die Ansicht 36 des Körperteils aus computertomographisch gewonnenen Daten angezeigt wird. Durch Betätigen des zweiten Steuerelementes 34.2 kann ein Anwender bewirken, dass die Ansicht 36 aus magnetresonanztomographisch gewonnenen Daten generiert wird. In jedem Fall löst das Betätigen eines jeweiligen Steuerelementes 34.1 oder 34.2 das Senden eines entsprechenden Steuersignals von der Bilddarstellungs- und Steuereinheit 30 zu der Datenverarbeitungs- und Bilderzeugungseinheit aus. Diese generiert dann entsprechende Ansichten 36 des Körperteils und überträgt diese zu der Bilddarstellungs- und Steuereinheit 30, auf deren Anzeige- und Eingabeeinheit 32 dann die entsprechende Ansicht 36 dargestellt wird.

Ein drittes Steuerelement 34.3 erlaubt die Aufnahme von Schnappschüssen entweder nur einer jeweils aktuellen Ansicht 36 des Körperteils oder auch der gesamten, auf der Anzeige- und Eingabeeinheit 32 abgebildeten Darstellung einschließlich der Steuerelemente. Wird das dritte Steuerelement 34.3 von einem Anwender betätigt, generiert die Bilddarstellungs- und Steuereinheit 30 ein Steuersignal, das die Datenverarbeitungs- und Bilderzeugungseinrichtung 20 dazu veranlasst, eine entsprechende Ansicht so zu speichern, dass sie später wieder abrufbar ist.

In ähnlicher Weise erlaubt ein viertes Steuerelement 34.4 das Auslösen der Aufnahme einer Bildfolge, die nach Art eines Films entweder auf der Bilddarstellungs- und Steuereinheit 30 selbst oder auch auf der Datenverarbeitungs- und Bilderzeugungseinrichtung 20 gespeichert wird. Bei einem jeweiligen erstmaligen Betätigen des vierten Steuerelementes 34.4 wird die Aufnahme einer solchen Bildfolge gestartet, während ein zweitmaliges Drücken des vierten Steuerelementes 34.4 die Aufnahme der Bildfolge wieder stoppt. Nach erstmaligen Betätigen des vierten Steuerelementes 34. 4 ändert sich dessen Darstellung, so dass ein Anwender sofort weiß, dass aktuell die Aufnahme einer Bildfolge erfolgt. Wird diese Aufnahme einer Bildfolge durch ein jeweiliges zweitmaliges Betätigen des vierten Steuerelementes 34.4 wieder gestoppt, wird das vierte Steuerelement 34.4 wieder in seiner in Figuren 3 und 4 abgebildeten ursprünglichen Darstellungsform dargestellt.

Weitere Steuerelemente 34.6 und 34.7 dienen beispielsweise der Einstellung von Kontrast (Steuerelement 34.6) und der Helligkeit (Steuerelement 34.7) der angezeigten Ansicht.

Ein achtes Steuerelement erlaubt einen Reset. Über Betätigung des achten Steuerelementes 34.8 werden die ursprünglichen Einstellungen wiederhergestellt.

Mit einem neunten Steuerelement 34.9 können Positionen in der dargestellten Ansicht 36 des Körperteils markiert werden. Ein zehntes Steuerelement 34.10 erlaubt die Umschaltung zwischen verschiedenen Darstellungsmaßstäben, wobei der Darstellungsmaßstab 1:1 bevorzugt ist.

Auch erlaubt die Bilddarstellungs- und Steuereinheit 30 das Umschalten zwischen verschiedenen Instrumenten, die drahtlos mit der Bilddarstellungs- und Steuereinheit 30 verbunden sind, beispielsweise zwischen Endoskop und Chirurgieinstrument.

Ein Umschalten zwischen verschiedenen Instrumenten kann aber auch durch Aufnahme eines jeweiligen Instrumentes durch einen Operateur automatisch erfolgen. In diesem Falle wird das jeweils in Benutzung befindliche Instrument durch ein Informationsicon wie das Icon 38.1 auf der Anzeige- und Eingabeeinheit 32 angezeigt.

Im Zusammenspiel der Bilddarstellungs- und Steuereinheit 30 mit einem Instrument 16 in Form eines Endoskopes ergibt sich eine besonders vorteilhafte Anwendung dahingehend, dass beispielsweise mit dem Endoskop bestimmte Bildpunkte beziehungsweise Positionen in einer zweidimensionalen Ebene oder in einem dreidimensionalen Raum ins Auge gefasst (d.h. beispielsweise ins Zentrum des Endoskop-Bildes gebracht) werden können. Durch Betätigen eines entsprechenden Steuerelementes entweder auf der Anzeige- und Eingabeeinheit 32 der Bilddarstellungs- und Steuereinheit 30 oder auch an dem Endoskop selbst kann eine jeweilige Position gespeichert werden. Mehrere solcher Positionen können dann anschließend zu einem Polygonenzug miteinander verbunden werden, der beispielsweise eine Körperhöhle oder auch einen operativ (beispielsweise nach Tumorentfernung) künstlich geschaffenen Hohlraum - genauer: dessen Umriss - beschreibt. Ein derartiger Polygonenzug kann dann als Schablone für das Ausschneiden von Körpergewebeteilen dienen, die beispielsweise zum Ausfüllen des künstlich geschaffenen Hohlraums dienen sollen und an anderer Stelle des Körpers eines Patienten entnommen werden.

An Stelle einen Polygonenzug aus einzelnen, mittels des Endoskops angefahrenen Körperpositionen zu generieren kann auch vorgesehen sein, die Ausrichtung des Endoskops kontinuierlich zu verfolgen, um so eine kontinuierlich aufgenommene Schablone zu generieren. Hierbei würden jedoch auch Fehlausrichtungen des Endoskops während der Aufnahme der Schablone mit aufgenommen werden.

Darüberhinaus erlaubt es auch die Bilddarstellungs- und Steuereinheit 30 selbst, dass ein Anwender auf der Ansicht 36 eines Körperteils beispielsweise mit einem Eingabestift einzelne Positionen markiert oder auch dargestellte Konturen nachfährt, um so die entsprechenden Positionsdaten für das Erzeugen einer Schablone zu generieren.

Eine weitere Funktion, die die Bilddarstellungs- und Steuereinheit 30 bietet, ist das Antasten von Landmarken auf der Ansicht 36 des Körperteils, um so beispielsweise verschiedene Darstellungen wie beispielsweise eine endoskopische Aufnahme und einer aus tomographisch gewonnenen Daten generierte Ansicht miteinander in Einklang zu bringen, den entsprechenden Landmarken in den verschiedenen Darstellungen einander zugeordnet werden.

Auch kann vorgesehen sein, dass sich die Bilddarstellungs- und Steuereinheit 30 mit verschiedenen Instrumenten und/oder Datenverarbeitungs- und Bilderzeugungseinrichtungen 20 verbinden lässt, so dass mittels einer einzigen Bilddarstellungs- und Steuereinheit 30 mehrere Operationen durch Umschalten zwischen verschiedenen Datenverarbeitungs- und Bilderzeugungseinrichtungen 20 verfolgt werden können.

Grundeinstellungen für verschiedene Benutzer einer Bilddarstellungs- und Steuereinheit 30 und/oder Grundeinstellungen für verschiedene Bilddarstellungs- und Steuereinheiten 30 können entweder auf einer jeweiligen Bilddarstellungs- und Steuereinheit 30 oder aber auch auf einer entsprechenden Datenverarbeitungs- und Bilderzeugungseinrichtung 20 gespeichert werden, insbesondere wenn Letzere in Form eines zentralen Servers oder Rechenzentrums verwirklicht ist. Dies erlaubt es, insbesondere auch auf einer zentralen Datenverarbeitungs- und Bilderzeugungseinrichtung 20 Grundeinstellungen für verschiedene Benutzer so zu speichern, dass jeweils unterschiedliche Bilddarstellungs- und Steuereinheiten 30 auch mit verschiedenen Betriebssystemen zum Einsatz zu kommen können, ohne dass sich ein Benutzer auf jeweils ganz andere Darstellungsweisen oder Voreinstelllungen einstellen muss. Vielmehr können die Darstellungsweisen und Voreinstellungen unabhängig von einer jeweils konkreten Bilddarstellungs- und Steuereinheit 30 und deren speziellen Betriebssystemen auf verschiedenen Bilddarstellungs- und Steuereinheiten jeweils gleich oder zumindest ähnlich realisiert werden.

Darüberhinaus ist es möglich, dass in einer jeweiligen Bilddarstellungs- und Steuereinheit 30 Daten hinterlegt sind, die ein jeweiliges medizinisches System entweder teilweise oder vollständig beschreiben. Dazu können Gerätebeschreibungen gehören genauso wie Beschreibungen der Lageerfassungseinrichtung oder aber auch der Datenverarbeitungs- und Bilderzeugungseinrichtung. Solche Daten können dauerhaft in einer jeweiligen Bilddarstellungs- und Steuereinheit 30 hinterlegt sein oder aber auch erst mit Beginn einer jeweiligen Anwendung, beispielsweise von einer entsprechenden Datenverarbeitungs- und Bilderzeugungseinrichtung 20 zu einer jeweiligen Bilddarstellungs- und Steuereinheit 30 übertragen werden. Im letztegenannten Fall können insbesondere auch unterschiedliche Bilddarstellungs- und Steuereinheit 20 unterschiedlicher Art oder Ausstattung leicht gegeneinander ausgetauscht werden.

Umgekehrt können auch in einer jeweiligen Datenverarbeitungs- und Bilderzeugungseinrichtung 20 Daten hinterlegt sein, die einzelne individuelle Bilddarstellungs- und Steuereinheiten 30 beschreiben, gegebenenfalls auch individuell für unterschiedliche Nutzer einer jeweiligen Bilddarstellungs- und Steuereinheit 30.

In einem Extrem kann auch die vollständige Datenverarbeitungs- und Bilderzeugungseinrichtung 20 in eine jeweilige Bilddarstellungs- und Steuereinheit 30 integriert sein.

Umgekehrt ist es auch möglich, dass alle wesentlichen Informationen in der Datenverarbeitungs- und Bilderzeugungseinrichtung 20 gespeichert sind, so dass zum Einen die Bilddarstellungs- und Steuereinheit 30 recht einfach gehalten werden kann und zum Anderen alle wesentlichen Informationen zentral verfügbar und damit auch für andere Benutzer oder Bilddarstellungs- und Steuereinheiten 30 verfügbar gehalten sind. Wenn beispielsweise die gesamte Darstellung auf einer jeweiligen Anzeige- und Eingabeeinheit 32 einer Bilddarstellungs- und Steuereinheit 30 von einer entsprechenden Datenverarbeitungs- und Bilderzeugungseinrichtung 20 einschließlich der Positionen und Darstellungsformen der Steuerelemente vorgegeben werden, kann es ausreichen, dass eine jeweilige Bilddarstellungs- und Steuereinheit 30 bei Berühren eines entsprechenden, dargestellten Steuerelementes auf der Anzeige- und Eingabeeinheit 32 nur noch ein Steuersignal generiert, welches die jeweils berührte Position auf der Anzeige- und Eingabeeinheit 32 wiederspiegelt. Diese Information reicht dann für die Datenverarbeitungs- und Bilderzeugungseinrichtung 20 aus, um zu erkennen, welches Steuerelement auf der Bilddarstellungs- und Steuereinheit 30 betätigt wurde oder welche Positionen auf der dargestellten Ansicht 36 eines Körperteils markiert wurde.

Indem auf diese Weise alle wesentlichen Funktionalitäten der Bilddarstellungs- und Steuereinheit 30 tatsächlich von der Datenverarbeitungs- und Bilderzeugungseinrichtung 20 bereitgestellt und ausgeführt werden, können gleichzeitige leistungsfähige Funktionalitäten bereitgestellt werden und einfache, insbesondere kleine und leichte Bilddarstellungs- und Steuereinheiten 30 bereitgestellt werden. Da sich die Datenverarbeitungs- und Bilderzeugungseinrichtung 20 wie bereits ausgeführt auch nicht in unmittelbarer Nähe eines Anwenders oder Operateurs befinden muss, sondern beispielsweise in einem zentralen Rechenzentrum realisiert sein kann, kann ein Anwender oder Operateur in seinem Arbeitsumfeld sich ganz auf seine Instrumente konzentrieren und gegebenenfalls Unterstützung auf die Bilddarstellungs- und Steuereinheit 30 zurückgreifen. Da diese gegebenenfalls auch als Fernsteuerung für die entsprechenden Instrumente des Anwenders oder Operateurs dienen kann (siehe oben) erleichtert dies dem Anwender und Operateur seiner Arbeit am Arbeitsplatz - beispielsweise im Operationssaal - sehr.

Insbesondere erlaubt eine hier beschriebene Bilddarstellungs- und Steuereinheit 30 ein instrumentenzentriertes Arbeiten des Anwenders oder Operateurs, bei dem dieser sich ganz auf seine Instrumente konzentrieren kann und von weiterem technischem Ballast weitestgehend befreit ist.

### Bezugszeichenliste

- 10: Patientenkopf
- 12: Feldgenerator
- 14: patientenfester Positionssensor
- 16: bewegliches Instrument
- 18: Handgriff des beweglichen Instruments
- 20: Datenverarbeitungs- und Bilderzeugungseinrichtung
- 22: Verbindungskabel
- 24: Instrumentenkabel
- 30: Bilddarstellungs- und Steuereinheit
- 32: Anzeige- und Eingabeeinheit
- 34: Steuerelement
- 36: Ansicht eines Körperteils
- 38: Informationsicon

## Patentansprüche

1. Medizinisches System mit einem Instrument (16), einer Lageerfassungseinrichtung (12, 14) für das Instrument, einer Datenverarbeitungs- und Bilderzeugungseinrichtung (20) und einer Bilddarstellungs- und Steuereinheit (30),
von denen die Lageerfassungseinrichtung (12, 14) ausgebildet ist, eine Position des Instruments (16) bezüglich eines Referenzkoordinatensystems zu erfassen und
von denen die Datenverarbeitungs- und Bilderzeugungseinrichtung (20) ausgebildet ist, aus aktuell aufgenommenen oder gespeicherten, ein Körperteil repräsentierenden Daten ein Bild des Körperteils zu erzeugen, das eine Ansicht (36) des Körperteils zusammen mit einer Darstellung der Position des Instruments (16) derart wiedergibt, dass ein Betrachter die Position des Instruments (16) in dem Körperteil dem Bild des Körperteils entnehmen kann,
wobei die Bilddarstellungs- und Steuereinheit (30) drahtlos mit der Datenverarbeitungs- und Bilderzeugungseinrichtung (20) verbunden und eine Anzeige- und Eingabeeinheit (32) mit geschlossener Oberfläche aufweist, auf der im Betrieb Steuerelemente (34) sowie eine jeweilige Ansicht (36) des Körperteils zusammen mit einer Darstellung der Position des Instruments (16) dargestellt werden, wobei die Bilddarstellungs- und Steuereinheit (30) weiterhin ausgebildet ist, über angezeigte Steuerelemente (34) Benutzereingaben entgegenzunehmen und in Abhängigkeit jeweils entgegengenommener Benutzereingaben Steuersignale an die der Datenverarbeitungs- und Bilderzeugungseinrichtung (20) zu senden,
**dadurch gekennzeichnet, dass** die Darstellungsweise betreffende Grundeinstellungen für unterschiedliche Bilddarstellungs- und Steuereinheiten auf der Datenverarbeitungs- und Bilderzeugungseinrichtung gespeichert sind.

2. Medizinisches System gemäß Anspruch 1, **dadurch gekennzeichnet, dass** die Datenverarbeitungs- und Bilderzeugungseinrichtung (20) weiterhin ausgebildet ist, in einer jeweiligen Ansicht (36) eines Körperteils neben einer jeweiligen Position des Instruments eine Markierung einer weiteren Position und/oder eines Zielgebietes oder Zielvolumens in dem Körperteil anzuzeigen.

3. Medizinisches System gemäß Anspruch 1 oder 2, **dadurch gekennzeichnet, dass** die Anzeige- und Eingabeeinheit (32) ausgebildet ist, eine jeweilige Ansicht (36) des Körperteils im Maßstab 1:1 darzustellen, so dass Entfernungen in einer dargestellten Schnittebene eines Körperteils einer Entfernung entlang der Schnittebene in dem Körperteil selbst wenigstens annähernd identisch sind.

4. Medizinisches System gemäß einem der Ansprüche 1 bis 3, **dadurch gekennzeichnet, dass** die Datenverarbeitungs- und Bilderzeugungseinrichtung (20) ausgebildet ist, auf unterschiedliche, ein jeweiliges Körperteil repräsentierende Daten zuzugreifen, wobei die Datenverarbeitungs- und Bilderzeugungseinrichtung (20) entsprechend dazu ausgebildet ist, nach Wahl ein Bild zu erzeugen, das auf jeweils ausgewählten, ein jeweiliges Körperteil repräsentierenden Daten basiert und wobei die Bilddarstellungs- und Steuereinheit (30) ausgebildet ist, eine oder mehrere Steuerelemente (34) darzustellen, die eine Auswahl zwischen verschiedenen, ein jeweiliges Körperteil repräsentierenden Daten und damit der entsprechenden Bilddarstellung erlauben, wobei die Bilddarstellungs- und Steuereinheit (30), bei Betätigung eines entsprechenden Steuerelementes (34) ein Steuersignal erzeugt, das die Datenverarbeitungs- und Bilderzeugungseinrichtung (20) dazu veranlasst, ein Bild auf Basis der entsprechend ausgewählten, ein Körperteil des Patienten repräsentierenden Daten zu generieren.

5. Medizinisches System gemäß einem der Ansprüche 1 bis 4, **dadurch gekennzeichnet, dass** die Datenverarbeitungs- und Bilderzeugungseinrichtung (20) ausgebildet ist, ein angezeigtes Bild so zu speichern, dass es später wieder aufgerufen werden kann, und dass die Bilddarstellungs- und Steuereinheit (30) ein entsprechendes Steuerelement (34) aufweist, bei dessen Betätigung die Bilddarstellungs- und Steuereinheit (30) ein Steuersignal erzeugt, das die Datenverarbeitungs- und Bilderzeugungseinrichtung (20) dazu veranlasst, ein bei Betätigung des Steuerelementes (34) jeweils aktuell angezeigtes Bild zu speichern.

6. Medizinisches System gemäß einem der Ansprüche 1 bis 5, **dadurch gekennzeichnet, dass** die Datenverarbeitungs- und Bilderzeugungseinrichtung (20) ausgebildet ist, eine Bildfolge nach Art eines Films zu speichern und dass die Bilddarstellungs- und Steuereinheit (30) ein entsprechendes Steuerelement (34) aufweist, bei dessen Betätigung die Bilddarstellungs- und Steuereinheit (30) ein Steuersignal erzeugt, das die Datenverarbeitungs- und Bilderzeugungseinrichtung dazu veranlasst, eine Bildfolge im Sinne eines Films aufzunehmen.

7. Medizinisches System gemäß Anspruch 6, **dadurch gekennzeichnet, dass** die Bilddarstellungs- und Steuereinheit (30) ausgebildet ist, eine Darstellung des Steuerelementes (34) zum Auslösen des Aufnehmens einer Bildfolge bei einer Betätigung, die das Aufnehmen einer Bildfolge startet, zu ändern.

8. Medizinisches System gemäß Anspruch 6 oder 7, **dadurch gekennzeichnet, dass** die Bilddarstellungs- und Steuereinheit (30) so ausgebildet ist, dass sie bei einem zweiten Betätigen des Steuerelementes (34) für die Aufnahme von Bildfolgen ein Steuersignal erzeugt, dass die Datenverarbeitungs- und Bilderzeugungseinrichtung (20) dazu veranlasst, die Aufnahme einer jeweiligen Bildfolge zu stoppen.

9. Medizinisches System gemäß Anspruch 7 und 8, **dadurch gekennzeichnet, dass** die Bilddarstellungs- und Steuereinheit (30) so ausgebildet ist, dass sie beim jeweils zweitmaligen Betätigen des Steuerelementes (34) wieder die ursprüngliche Darstellung dieses Steuerelementes (34) vor dem Auslösen der Aufnahme einer Bildfolge zur Anzeige bringt.

10. Medizinisches System gemäß wenigstens einem der Ansprüche 1 bis 9, **dadurch gekennzeichnet, dass** die Bilddarstellungs- und Steuereinheit (30) derart ausgebildet ist, dass ein Anwender auf einer jeweiligen Ansicht (36) eines Körperteils Landmarken antasten kann, um verschiedene Darstellungen wie eine endoskopische Aufnahme und eine aus tomographisch gewonnenen Daten generierte Ansicht miteinander so in Einklang zu bringen, dass entsprechende Landmarken in den verschiedenen Darstellungen einander zugeordnet sind.

## Claims

1. A medical system comprising an instrument (16), a position acquisition apparatus (12, 14) for the instrument, a data processing and image generating apparatus (20) and an image display and control unit (30),
of which the position acquisition apparatus (12, 14) is embodied to acquire a position of the instrument (16) in relation to a reference coordinate system and
of which the data processing and image generating apparatus (20) is embodied to generate an image of a body part from currently recorded or stored data representing a body part, which image reproduces a view (36) of the body part together with a representation of the position of the instrument (16) in such a way that an observer can gather the position of the instrument (16) in the body part from the image of the body part,
wherein the image display and control unit (30) is wirelessly connected to the data processing and image generating apparatus (20), and comprises a display and input unit (32) with a closed surface, on which control elements (34) and a respective view (36) of the body part are displayed during operation, together with a representation of the position of the instrument (16), wherein the image display and control unit (30) is furthermore embodied to accept user inputs via displayed control elements (34) and to transmit control signals to those of the data processing and image generating apparatus (20) as a function of the respectively accepted user inputs,
**characterized in that** basic settings concerning the representation are stored for different image display and control units on the data processing and image generating apparatus.

2. The medical system as claimed in claim 1, **characterized in that**, in addition to a respective position of the instrument, the data processing and image generating apparatus (20) is furthermore embodied to display in a respective view (36) of a body part a marking of a further position and/or of a target region or target volume in the body part.

3. The medical system as claimed in claim 1 or 2, **characterized in that** the display and input unit (32) is embodied to display a respective view (36) of the body part with a 1:1 scale such that distances in a depicted slice plane of a body part are at least approximately identical to a distance along the slice plane in the body part itself.

4. The medical system as claimed in one of claims 1 to 3, **characterized in that** the data processing and image generating apparatus (20) is embodied to access different data representing a respective body part, wherein the data processing and image generating apparatus (20) is accordingly embodied to generate an image, as desired, which image is based on respectively selected data representing a respective body part, and wherein the image display and control unit (30) is embodied to display one or more control elements (34), which permit a selection to be made between various data representing a respective body part and, hence, the corresponding image display, wherein the image display and control unit (30) generates a control signal when an appropriate control element (34) is actuated, which control signal causes the data processing and image generating apparatus (20) to generate an image on the basis of the correspondingly selected data representing a body part of the patient.

5. The medical system as claimed in one of claims 1 to 4, **characterized in that** the data processing and image generating apparatus (20) is embodied to store a displayed image in such a way that it can be recalled at a later time, and **in that** the image display and control unit (30) comprises a corresponding control element (34), wherein the image display and control unit (30) generates a control signal when said control element is actuated, which control signal causes the data processing and image generating apparatus (20) to store a respectively currently displayed image when the control element (34) is actuated.

6. The medical system as claimed in one of claims 1 to 5, **characterized in that** the data processing and image generating apparatus (20) is embodied to store an image sequence in the style of a film and **in that** the image display and control unit (30) comprises a corresponding control element (34), wherein the image display and control unit (30) generates a control signal when said control element is actuated, which control signal causes the data processing and image generating apparatus to record an image sequence within the meaning of a film.

7. The medical system as claimed in claim 6, **characterized in that** the image display and control unit (30) is embodied to modify a display of the control element (34) for triggering the recording of an image sequence in the case of an actuation which starts the recording of an image sequence.

8. The medical system as claimed in claim 6 or 7, **characterized in that** the image display and control unit (30) is embodied in such a way that it generates a control signal in the case of a second actuation of the control element (34) for recording image sequences, which control signal causes the data processing and image generating apparatus (20) to stop the recording of a respective image sequence.

9. The medical system as claimed in claim 7 and 8, **characterized in that** the image display and control unit (30) is embodied in such a way that, in the case of the respective second actuation of the control element (34), it once again brings to display the original display of this control element (34) prior to triggering the recording of an image sequence.

10. The medical system as claimed in at least one of the claims 1 to 9, **characterized in that** the image display and control unit (30) is embodied in such a way that a user can touch landmarks in a respective view (36) of a body part, in order to bring different representations such as an endoscopic image in conformity with a view rendered from tomographical data, in conformity with each other, such that respective landmarks in such different representations are assigned to each.

## Revendications

1. Système médical, comprenant un instrument (16), un dispositif (12, 14) de détection de la position de l'instrument, un dispositif (20) de traitement de données et de production d'image et une unité (30) de représentation d'image et de commande,
parmi lesquels le dispositif (12, 14) de détection de la position est constitué pour détecter une position de l'instrument (16) par rapport à un système de coordonnées de référence et
parmi lesquels le dispositif (20) de traitement de données et de production d'image est constitué pour produire, à partir de données enregistrées instantanément ou mises en mémoire et représentant une partie du corps, une image de la partie du corps, qui représente une vue (36) de la partie du corps, ensemble avec une représentation de la position de l'instrument (16), de manière à ce qu'un observateur puisse déduire, de l'image de la partie du corps, la position de l'instrument (16) dans la partie du corps,
dans lequel l'unité (30) de représentation d'image et de commutation est reliée sans fil au dispositif (20) de traitement de données et de production d'image et a une unité (32) d'affichage et d'entrée à surface fermée, sur laquelle sont représentés, en fonctionnement, des éléments (34) de commande, ainsi qu'une vue (36) de la partie du corps, ensemble avec une représentation de la position de l'instrument (16), l'unité (30) de représentation d'image et de commande étant constituée, en outre, pour recevoir des entrées d'utilisateur et pour, en fonction des entrées d'utilisateur reçues, envoyer des signaux de commande au dispositif (20) de traitement de données et de production d'image,
**caractérisé en ce que** les réglages de base, concernant le mode de représentation, des unités différentes de représentation d'image et de commande sont mis en mémoire sur le dispositif de traitement de données et de production d'image.

2. Système médical suivant la revendication 1, **caractérisé en ce que** le dispositif (20) de traitement de données et de production d'image est constitué, en outre, pour afficher, dans une vue (36) respective d'une partie du corps, outre une position respective de l'instrument, un repère d'une autre position et/ou d'un domaine cible ou d'un volume cible de la partie du corps.

3. Système médical suivant la revendication 1 ou 2, **caractérisé en ce que** l'unité (32) d'affichage et d'entrée est constituée pour représenter une vue (36) respective de la partie du corps à l'échelle 1:1, de manière à ce que des distances, dans un plan de coupe représenté d'une partie du corps, soient au moins à peu près identiques à une distance suivant le plan de coupe dans la partie du corps elle-même.

4. Système médical suivant l'une des revendications 1 à 3, **caractérisé en ce que** le dispositif (20) de traitement de données et de production d'image est constitué pour accéder à des données différentes représentant une partie du corps respective, le dispositif (20) de traitement de données et de production d'image étant constitué de manière correspondante, pour produire, au choix, une image, qui repose sur des données sélectionnées représentant une partie du corps, et dans lequel l'unité (30) de représentation d'image et de commande est constituée pour représenter un ou plusieurs éléments (34) de commande, qui permettent un choix entre diverses données représentant une partie du corps et ainsi la représentation d'image correspondante, l'unité (30) de représentation d'image et de commande produisant, lorsqu'un élément (34) de commande correspondant est actionné, un signal de commande, qui fait que le dispositif (20) de traitement de données et de production d'image produit une image sur la base des données sélectionnées de manière correspondante et représentant une partie du corps du patient.

5. Système médical suivant l'une des revendications 1 à 4, **caractérisé en ce que** le dispositif (20) de traitement de données et de production d'image est constitué pour mettre une image affichée en mémoire, de manière à pouvoir la rappeler ultérieurement et **en ce que** l'unité (30) de représentation d'image et de commande a un élément (34) de commande correspondant à l'actionnement duquel l'unité (30) de représentation d'image et de commande produit un signal de commande, qui fait que l'unité (20) de traitement de données et de production d'image met, à l'actionnement de l'élément (34) de commande, en mémoire respectivement une image affichée instantanément.

6. Système médical suivant l'une des revendications 1 à 5, **caractérisé en ce que** le dispositif (20) de traitement de données et de production d'image est constitué pour mettre en mémoire une séquence d'image à la manière d'un film et **en ce que** l'unité (30) de représentation d'image et de commande a un élément (34) de commande correspondant à l'actionnement duquel l'unité (30) de représentation d'image et de commande produit un signal de commande, qui fait que le dispositif de traitement de données et de production d'image enregistre une séquence d'image au sens d'un film.

7. Système médical suivant la revendication 6, **caractérisé en ce que** l'unité (30) de production d'image et de commande est constituée pour modifier une représentation de l'élément (34) de commande, afin de déclencher l'enregistrement d'une séquence d'image lors d'un actionnement, qui fait débuter l'enregistrement d'une séquence d'image.

8. Système médical suivant la revendication 6 ou 7, **caractérisé en ce que** l'unité (30) de représentation d'image et de commande est constituée de manière à produire, lors d'un deuxième actionnement de l'élément (34) de commande de l'enregistrement de séquence d'image, un signal de commande, de manière à ce que le dispositif (20) de traitement de données et de production d'image fasse cesser l'enregistrement d'une séquence d'image.

9. Système médical suivant la revendication 7 et 8, **caractérisé en ce que** l'unité (30) de représentation d'image et de commande est constituée de manière à, lors de l'actionnement pour la deuxième fois de l'élément (34) de commande, remettre en affichage la représentation d'origine de cet élément (34) de commande, avant le déclenchement de l'enregistrement d'une séquence d'image.

10. Système médical suivant au moins l'une des revendications 1 à 9, **caractérisé en ce que** l'unité (30) de représentation d'image et de commande est constituée de manière à ce qu'un utilisateur puisse, sur une vue (36) respective d'une partie du corps, repérer des repères pour mettre diverses représentations, comme un enregistrement endoscopique et une vue produite à partir de données obtenues par tomographie, en accord entre elles, de manière à associer entre eux des repères correspondants dans les diverses représentations.
